# FASCICULE DE BREVET EUROPEEN

(11) **EP 0 424 261 B1**
(45) Date de publication et mention de la délivrance du brevet: **03.01.1996**
(21) Numéro de dépôt: 90402928.7
(22) Date de dépôt: 18.10.1990
(51) Int. Cl.: A61K 7/13

(54) **Composition tinctoriale contenant des précurseurs de colorants par oxydation et des coupleurs amino indoliques**
Färbemittel, das Oxydationsfarbvorstufen und Aminoindol-Kuppler enthält
Dyeing composition containing oxidation dyes precursors and aminoindole derivatives as coupling agent

(30) Priorité: 20.10.1989 LU 87611
(43) Date de publication de la demande: 24.04.1991
(73) Titulaire: L'OREAL, F-75008 Paris (FR)
(72) Inventeur: Junino, Alex, F-93180 Livry-Gargan (FR); Lang, Gérard, F-95210 Saint-Gratien (FR); Cotteret, Jean, F-78480 Verneuil sur Seine (FR); Vandenbossche, Jean-Jacques, F-93600 Aulnay-sous-Bois (FR)
(74) Mandataire: Casalonga, Axel

(56) Documents cités:
- EP-A- 0 186 367
- EP-A- 0 271 186
- DE-A- 1 916 139
- US-A- 4 013 404
- CHEMICAL ABSTRACTS, vol. 91, 1979, page 641, résumé no. 140665z, Columbus,Ohio, US; A.N. KOST et al.: "Synthesis of aminoindoles by the Buchererreaction", & KHIM. GETEROTSIKL.SOEDIN. 1979, (6), 786-9
- J. CHEM. SOC., CHEM. COMMUN., vol. 23, 1982, pages 1356-1357, Londres, GB; S.V. LEY et al.: "An unexpected rearrangement of 4-alkylaminoindoles"

## Description

La présente invention est relative à de nouvelles compositions tinotoriales pour fibres kératiniques et en particulier pour cheveux humains. contenant des précurseurs de colorants d'oxydation et des coupleurs amino indoliques, et procédés de teinture mettant en oeuvre de telles compositions.

Il est connu de teindre les fibres kératiniques et en particulier les cheveux humains, avec des compositions tinotoriales contenant des précurseurs de colorants d'oxydation, en particulier des p-phénylène diamines, des ortho- ou para-aminophénols qu'on appelle généralement "bases d'oxydation".

On sait également qu'on peut faire varier les nuances obtenues avec ces bases d'oxydation en utilisant, en association avec ces bases, des coupleurs encore appelés modificàteurs de coloration choisis plus particulièrement parmi les métadiamines aromatiques, les méta-aminophénols et les métadiphénols.

Le brevet DE-A-1 916 139 décrit des compositions tinctoriales contenant des précurseurs de colorant d'oxydation de type ortho ou para et, en tant que coupleurs, des dérivés indolines.

Le brevet USP 4 013 404 (PARENT) décrit l'utilisation en tant que colorant d'oxydation de certains aminoindoles seuls ou associés à des coupleurs.

On recherche, dans le domaine de la teinture capillaire, des précurseurs de colorants d'oxydation ou des coupleurs permettant de conferer aux cheveux, en milieu alcalin oxydant généralement utilise en teinture d'oxydation, une coloration ayant une résistance satisfaisante à la lumière, aux lavages, aux intempéries et à la transpiration. On souhaite également obtenir des couleurs avec des reflets, ce qui, en teinture d'oxydation, nécessite l'emploi de colorants directs stables en milieu réducteur.

La demanderesse vient de découvrir de manière suprenante, ce qui fait l'objet de l'invention, que l'utilisation de certains dérivés indoliques à titre de coupleurs, avec des précurseurs de colorants d'oxydation de type para ou ortho, permettait d'obtenir après application sur les fibres kératiniques et en particulier les cheveux, des couleurs avec reflets présentant des résistances à la lumière, aux lavages, aux intempéries et à la transpiration, particulairement remarquables.

Un objet de l'invention est constitue par des compositions tinctoriales d'oxydation, destinées à être utilisées pour la teinture des fibres kératiniques; contenant au moins un précurseur de colorant d'oxydation de type para, le 6-méthyl 1-hydroxy 2-aminobenzène et/ou le 4-méthyl 1-amino 2-hydroxybenzène. avec certains dérivés amino indoliques définis ci-après.

Un autre objet ce l'invention est constitué par les procédés de coloration des fibres kératiniques, en particulier des cheveux humains, mettant en oeuvre une telle composition.

D'autres objets de l'invention apparaîtront à la lecture ce la description et des exemples qui suivent.

La composition tinctoriale d'oxydation conforme a l'invention, destinée à être utilisée pour la teinture des fibres kératiniques, en particulier des fibres kératiniques humaines et notamment des cheveux, est essentiellement caractérisée par le fait qu'elle contient dans un milieu solvant approprie pour la teinture de ces fibres, au moins un précurseur de colorant d'oxydation de type para le 6-méthyl 1-hydroxy 2-aminobenzène et/ou le 4-méthyl 1-amino 2-hydroxybenzène. et au moins un coupleur hétérocyclique, répondant à la formule (I) : 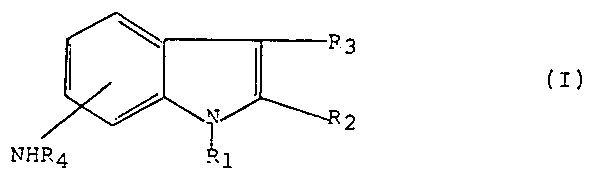
dans laquelle :
R₁ et R₃, identiques ou différents, désignent un atome d'hydrogène, un groupement alkyle en C₁-C₄;
R₂ désigne un atome d'hydrogène, un groupement alkyle en C₁-C₄, un groupement carboxyle ou un groupement alcoxycarbonyle; au moins l'un des radicaux R₂ ou R₃ désigne hydrogène;
R₄ désigne un atome d'hydrogène ou un groupement alkyle en C₁-C₄ ou hydroxyalkyle en C₁-C₄ ou polyhydroxyalkyle en C₂-C₄;
le groupement NHR₄ occupant les positions 4, 6 ou 7 du noyau benzénique, ainsi que leurs sels.

Parmi les composes de formule (I), les composés préférés sont ceux dans lesquels le radical alkyle désigne méthyle, éthyle, le radical hydroxyalxyle désigne hydroxyéthyle, le radical alcoxycarbonyle désigne méthoxy- ou éthoxycarbonyle.

Parmi les composes de formule (I), ceux repondant à la formule (IA) sont nouveaux et ceci constitue un autre objet de l'invention. 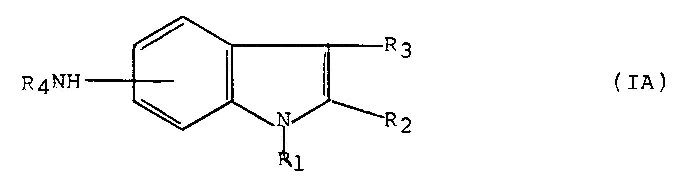
dans laquelle R₁, R₂, R₃ ont les significations indiquées ci-dessus et R₄ désigne alkyle en C₁-C₄, hydroxyalkyle en C₁-C₄, polyhydroxyalkyle en C₂-C₄, R₄NH occupant les positions 4, 6 ou 7 et R₄ étant différent d'alkyle lorsque R₄NH est en position 4.

Ces composés de formule (IA) peuvent être préparés selon le procédé suivant.

Le composé (IA) est obtenu à partir du composé (IB) (R₄=H) par les méthodes de substitution des amines aromatiques, selon le schéma réactionnel : 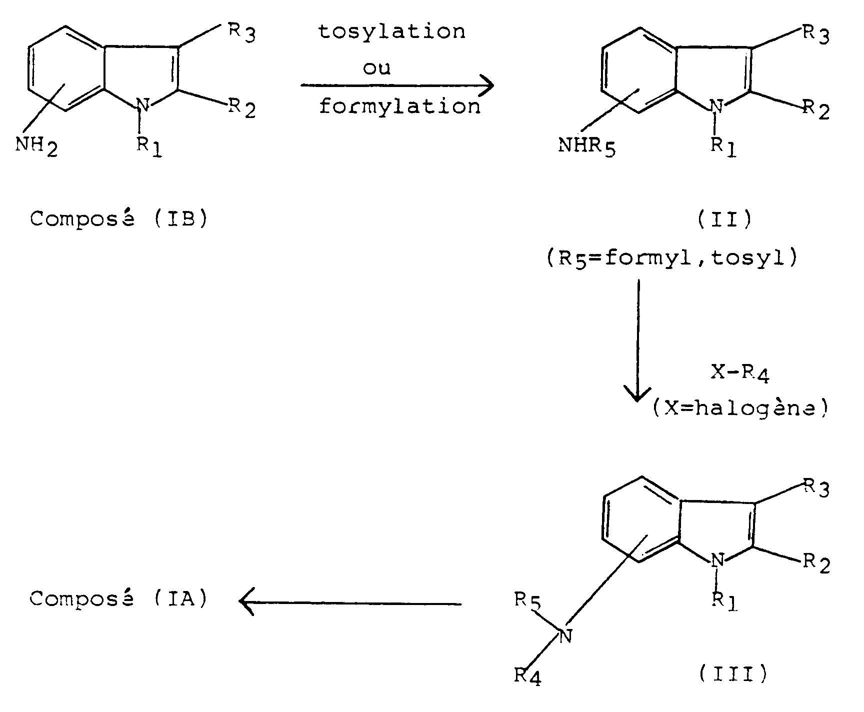
Par formylation ou tosylation, on obtient le compose (II). Le composé (II) est alkyle dans un deuxième temps par l'halogénure d'alkyle X-R₄. Lorsque l'halogénure d'alkyle est utilise en exces, un second groupement R₄ est introduit. Le produit attendu (IA) est obtenu par déformylation ou détosylation du composé (III), selon les méthodes conventionnelles.

Parmi les méthodes d'hydroxyéthylation, on peut citer l'action du chloroformiate de β-chloréthyle sur le composé (IB) qui permet d'obtenir dans un premier temps le carbamate de β-chloréthyle correspondant qui, soumis dans un deuxième temps à l'action d'une base minérale forte, permet d'obtenir le compose (IA) pour lequel le radical R₄ est un radical β-hydroxyéthyle.

Parmi les composés de formule (I), on peut citer le 6-amino indole, le 7-amino indole, le 6-N-β-hydroxyéthylamino indole, le 6-N-β-hydroxy-éthylamino 1-méthyl indole, le 6-méthylamino indole, le 6-amino N-méthyl indole, le 6-amino 2-carboxy indole, le 6-amino 3-méthyl indole, le 6-amino 2-méthyl indole, le 6-amino 2-éthoxycarbonyl indole, le 6-N(β, γ-dihydroxypropyl)amino indole.

Parmi ces composes, on notera que, par couplage oxydatif avec les précurseurs de colorants définis ci-après, le 6-amino indole et ses dérivés conduisent à des nuances à dominantes cuivrées (nuances chaudes); et que le 7-amino indole et ses dérivés conduisent à des nuances à reflets cendres ou acajou.

Les précurseurs de colorants de type para ou le 6-méthyl 1-hydroxy 2-aminobenzène et le 4-méthyl 1-amino 2-hydroxybenzène sont des composés qui ne sont pas des colorants en eux-mêmes, mais qui forment des colorants par un processus de condensation oxydative, soit sur eux-mêmes, soit en présence d'un coupleur ou modificateur.

Ces précurseurs de colorants de type para sont des composés benzéniques ou hétérocycliques qui comportent deux groupements amino ou bien un groupement amino et un hydroxyle en position para l'un par rapport à l'autre.

Ces précurseurs de colorants de type para peuvent être choisis parmi les paraphénylènediamines, les para-aminophénols, les précurseurs hétérocycliques para dérivés de la pyridine ou de la pyrimidine, tels que la 2,5-diaminopyridine, la 2-hydroxy 5-aminopyridine, la 2,4,5,6-tétraaminopyrimidine, des bases dites "doubles".

A titre de paraphénylènediamines, on peut plus particulièrement citer les composés répondant à la formule (II) ci-après : 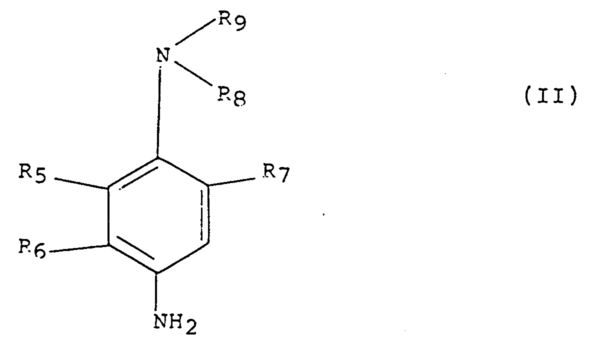
dans laquelle :
R₅, R₆ et R₇, identiques ou différents, représentent un atome d'hydrogène ou d'halogène, un radical alkyle, un radical alcoxy;
R₈ et R₉, identiques ou différents, représentent un atome d'hydrogène, un radical alkyle, hydroxyalkyle, alcoxyalkyle, carbamylalkyle, mésylamino alkyle, acétylaminoalkyle, uréidoalkyle, carbalcoxyamino alkyle, pipéridinoalkyle, morpholinoalkyle; ou bien R₈ et R₉ forment, conjointement avec l'atome d'azote auquel ils sont liés, un hétérocycle pipéridino ou morpholino, sous reserve que R₅ ou R₇ représente un atome d'hydrogène lorsque R₈ et R₉ ne représentent pas un atome d'hydrogène;
ainsi que les sels de ces composés.

Les bases dites doubles sont des bis-phénylalkylènediamines, répondant à la formule : 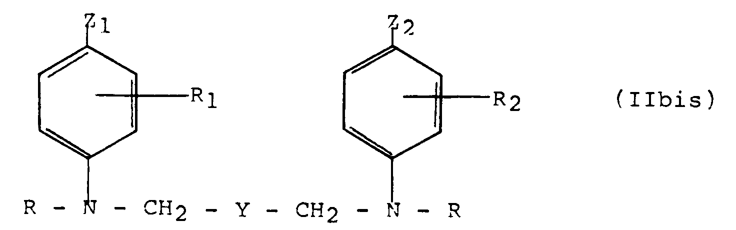
dans laquelle :
Z₁ et Z₂, identiques ou différents, représentent des groupements hydroxyle ou NHR₃, où R₃ désigne un atome d'hydrogène ou un radical alcoyle inférieur;
R₁ et R₂, identiques ou différents, représentent soit des atomes d'hydrogène, soit des atomes d'halogène, soit encore des groupements alcoyle;
R représente un atome d'hydrogène, un groupe alcoyle, hydroxyalcoyle ou aminoalcoyle, dont le reste amino peut être substitué;
Y représente un radical pris dans le groupe constitué par les radicaux suivants : -(CH₂)ₙ-, (CH₂)ₙ, -O-(CH₂)ₙ,-, -(CH₂)ₙ,-CHOH- (CH₂)ₙ,-(CH₂)ₙ, 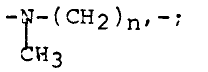
n étant un nombre entier compris entre 0 et 8 et n′ un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (II), on peut citer en particulier la p-phénylènediamine, la p-toluylènediamine, la méthoxyparaphénylènediamine, la 2,6-diméthyl p-phénylènediamine, la 2,5-diméthyl p-phénylènediamine, la 2-méthyl 5-méthoxy paraphénylènediamine, la 2,6-diméthyl 5-méthoxy p-phénylènediamine, la N,N-diméthylparaphénylènediamine, la 3-méthyl 4-amino N,N-diéthylaniline, la N,N-di(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthylcarbamylméthyl)aniline, la 3-méthyl 4-amino N,N-(éthylcarbamylméthyl)aniline, la 4-amino N,N-(éthyl β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline,la N-[(4′-amino)phényl]morpholine, la N-[(4′-amino)phényl]pipéridine. Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxy méthyl 4-aminophénol, le 2-(β-hydroxyéthyl)4-aminophénol, le 2-méthoxy 4-aminophénol, le 3-méthoxy 4-aminophénol, le 2-méthoxyméthyl 4-aminophénol.

Parmi les composés de formule (IIbis), on peut citer le N,N'bis-(β-hydroxyéthyl)N,N'-bis(4'-amino phenyl)1,3-diamino 2-propanol, la N,N'bis-(β-hydroxyethyl)N,N'-bis(4'-aminophenyl)éthylènediamine, la N,N' bis-(4-aminophenyl)tétraméthylènediamine, la N,N'bis(β-hydroxyéthyl)N,N'bis(4-aminophényl)tétraméthylène diamine, la N,N'bis-(4-méthylaminophényl)tétraméthylène diamine, la N,N'bis(éthyl)N,N'-bis(4'-amino 3'-methyl-phényl)éthylènediamine.

Les compositions tinctoriales peuvent egalement contenir en plus du coupleur hétérocyclique répondant à la formule (I) ci-dessus, d'autres coupleurs connus en eux-mêmes, tels que les métadiphenols, les métaaminophénols, les métaphénylènediamines, les métaacylaminophénols, les métauréidophénols, les métacarbalcoxyaminophénols, l'α-naphtol, les coupleurs possédant un groupe méthylène actif, tels que les composés β-cétoniques et les pyrazolones.

On peut citer plus particulièrement, à titre d'exemple, le 2,4-dihydroxy phénoxyéthanol, le 2,4-dihydroxy anisole, le métaaminophénol, le monométhyl éther de résorcine, le 2-méthyl 5-aminophénol, le 2-méthyl 5-N-(β-hydroxyéthyl)aminophénol, le 2-méthyl 5-N-(β-mésylaminoéthyl)aminophénol, le 2,6-diméthyl 3-aminophénol, la 6-hydroxybenzomorpholine, le 2,4-diamino anisole, le 2,4-diaminophénoxyéthanol, la 6-aminobenzomorpholine, le 2-[N-(β-hydroxyéthyl)amino 4-amino]phénoxyéthanol, le 2-amino 4-N-(β-hydroxyéthyl) amino anisole, le (2,4-diamino)phényl,β, γ-dihydroxy propyléther, la 2,4-diaminophénoxyéthylamine, et leurs sels.

On peut rajouter à ces compositions, comme cela est bien connu dans l'état de la technique, des colorants directs, tels que des colorants azoïques, anthraquinoniques ou les dérivés nitrés de la série benzénique.

L'ensemble des précurseurs de colorants par oxydation de type para, le 6-méthyl 1-hydroxy 2-aminobenzène et/ou le 4-méthyl 1-amino 2-hydroxybenzène. ainsi que les coupleurs utilises dans les compositions tinctoriales conformement à l'invention, représente de préférence de 0,3 à 7% en poids par rapport au poids total de la composition. La concentration en composes (I) peut varier entre 0,05 et 3,5% en poids par rapport au poids total de la composition.

Le milieu solvant approprie pour la teinture est généralement aqueux et son pH peut varier entre 8 et 11 et il est de préférence compris entre 9 et 11.

Il est ajuste à la valeur désirée à l'aide d'agents alcalinisants bien connus dans l'état de la technique, tels que l'ammoniaque, les carbonates alcalins, les alcanolamines comme la mono-, la di- ou la triéthanolamine.

Les compositions tinctoriales conformes à l'invention contiennent également dans leur forme de réalisation préférée, des agents tensio-actifs anioniques, cationiques, non ioniques, amphotères ou leurs mélanges. Parmi ces agents tensio-actifs, on peut citer les alkylbenzènesulfonates, les alkylnaphtalène sulfonates, les sulfates, les éthersulfates et les sulfonates d'alcools gras, les sels d'ammonium quaternaires, tels que le bromure de triméthylcétyl ammonium, le bromure de cétylpyridinium, les éthanolamides d'acides gras éventuellement oxyéthylénés, les acides, les alcools ou les amines polyoxyéthylénés, les alcools polyglycérolés, les alkylphènols polyoxy éthylénés ou polyglycérolés, ainsi que les alkylsulfates polyoxyéthylénés.

Ces agents tensio-actifs sont présents dans les compositions conformes à l'invention dans des proportions comprises entre 0,5 et 55% en poids, et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols, comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues ou leurs mélanges.

Les solvants sont présents de préférence dans une proportion comprise entre 1 et 40% en poids, et en particulier entre 5 et 30% en poids par rapport au poids total de la composition.

Les agents épaississants que l'on peut ajouter dans les compositions conformes à l'invention peuvent être choisis parmi l'alginate de sodium, la gomme arabique ou la gomme de guar, les dérivés de cellulose, tels que la méthylcellulose, l'hydroxyéthylcellulose, l'hydroxypropylcellulose, l'hydroxyméthylcellulose, la carboxyméthylcellulose, les hétérobiopolysaccharides, tels que la gomme de xanthane, les polymères dérivés d'acide acrylique. On peut également utiliser des agents épaississants minéraux, tels que la bentonite. Ces agents épaississants sont présents de préférence dans des proportions comprises entre 0,1 et 5% et en particulier entre 0,2 et 3% en poids par rapport au poids total de la composition.

Les agents antioxydants qui peuvent être présents dans les compositions sont choisis en particulier parmi le sulfite de sodium, l'acide thioglycolique, le bisulfite de sodium, l'acide ascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des tampons, des parfums, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment celle des cheveux humains. Ces compositions peuvent être conditionnées en flacons aerosols en présence d'un agent propulseur et former des mousses.

Les compositions tinctoriales conformes a l'invention et contenant un précurseur de colorant d'oxydation de type para, le 6-méthyl 1-hydroxy 2-aminobenzène ou/et le 4-méthyl 1-amino 2-hydroxybenzène. et un coupleur de formule (I), sont utilisées suivant un procédé mettant en oeuvre la révélation par un agent oxydant.

Conformément à ce procédé, on mélange au moment de l'emploi, la composition tinctoriale décrite ci-dessus avec une solution oxydante dans une quantite suffisante pour pouvoir développer une coloration, puis on applique le mélange obtenu sur les fibres kératiniques et en particulier les cheveux humains.

La solution oxydante contient des agents oxydants, tels que l'eau oxygénée, le peroxyde d'urée ou des persels, tels que le persulfate d'ammonium. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on rince les cheveux, on les lave au shampooing, on les rince à nouveaux et on les sèche.

Le coupleur hétérocyclique de formule (I) défini ci-dessus peut également être mis en oeuvre dans un procédé à plusieurs étapes, consistant dans l'une des étapes, à appliquer un précurseur de colorant d'oxydation para, le 6-méthyl 1-hydroxy 2-aminobenzène et/ou le 4-méthyl 1-amino 2-hydroxy benzène et, dans une autre étape, à appliquer le coupleur de formule (I).

L'agent oxydant peut être introduit, tout juste avant l'application, dans la composition appliquée dans le deuxième temps ou bien être additionnée sur les fibres kératiniques elles-mêmes dans un troisième temps, les conditions de pose et de séchage ou de lavage étant identiques.

La présente invention a également pour objet l'utilisation d'un composé hétérocyclique de formule (I) défini ci-dessus, à titre de coupleur dans la teinture d'oxydation des fibres kératiniques mettant en oeuvre des précurseurs de colorants d'oxydation de type para ou ortho tels que le 6-méthyl 1-hydroxy 2-aminobenzène et le 4-méthyl 1-amino 2-hydroxybenzène.

Les exemples suivants sont destinés à illustrer l'invention.

### EXEMPLE DE PREPARATION 1

### Préparation du 6-N-β-hydroxyéthylamino indole.

### Etape 1

### Préparation du 6-N-(β-chloroéthoxycarbonyl)amino indole

On chauffe au reflux 0,05 mole (6,6 g) de 6-amino indole, 5,5 g de carbonate de calcium dans 30 ml de dioxane. On ajoute peu à peu 0,055 mole (7,9 g) de chloroformiate de β-chloréthyle. Le mélange réactionnel est dilué à la glace. Le produit attendu précipite. Il fond à 134°C.

L'analyse du produit recristallisé de l'éthanol donne les résultats suivants :

| Analyse pour C₁₁H₁₁N₂O₂Cl | | | | | |
|---|---|---|---|---|---|
| | C | H | Cl | O | N |
| Calculé | 55,36 | 4,65 | 14,85 | 13,41 | 11,74 |
| Trouvé | 55,40 | 4,68 | 14,72 | 13,27 | 11,67 |

### Etape 2

### Préparation du 6-N-β-hydroxyéthylamino indole.

On ajoute 0,28 mole (66,5 g) de 6-N-(β-chloroéthoxycarbonyl)amino indole à 200 ml de soude 4N et 66,5 ml d'éthanol. Le mélange réactionnel est chauffé 1 heure au reflux. On précipite le produit attendu par ajout de glace. Il fond à 99°C.

L'analyse élémentaire du produit obtenu donne les résultats suivants :

| Analyse pour C₁₀H₁₂N₂O | | | | |
|---|---|---|---|---|
| | C | H | N | O |
| Calculé | 68,16 | 6,86 | 15,90 | 9,08 |
| Trouvé | 67,88 | 6,91 | 15,91 | 9,15 |

### EXEMPLE DE PREPARATION 2

### Préparation du chlorhydrage de 6-N(β-hydroxyéthyl)amino 1-méthyl indole.

### Etape 1

### Préparation de la N-(6-indolyl)oxazolidine-1,3 one-2

A 120 ml d'une solution de méthylate de sodium à 30% dans le méthanol, on ajoute 60 ml de méthanol puis, sous agitation, 0,25 mole (60 g) de 6-(β-chloréthoxycarbonyl)amino indole (préparé selon la première étape de l'exemple 1). La température atteint 50°C. L'agitation est maintenue 15 minutes après la fin de l'addition. Le précipité formé est essoré, lavé à l'alcool puis séché. Il fond à 199°C.

L'analyse du produit recristallisé de l'acide acétique donne les résultats suivants :

| Analyse pour C₁₁H₁₀N₂O₂ | | | | |
|---|---|---|---|---|
| | C | H | N | O |
| Calculé | 65,34 | 4,98 | 13,85 | 15,82 |
| Trouvé | 65,42 | 5,02 | 13,75 | 15,86 |

### Etape 2

### Préparation de la N-[6-(1-méthyl)indolyl]oxazolidine-1,3 one-2

A une solution de 0,15 mole (30,5 g) de N-(6-indolyl)oxazolidine-1,3 one-2 dans 300 ml de diméthylformamide, on ajoute 100 ml d'une solution à 30% de méthylate de sodium dans le méthanol. Le mélange réactionnel est chauffé à 40°C. On ajoute goutte à goutte 28 ml d'iodure de méthyle. Le chauffage est maintenu 1 heure après la fin de l'addition. Après dilution du milieu réactionnel par de l'eau glacée, le produit attendu précipite. Après essorage, lavage à l'eau puis à l'éthanol, il fond à 160°C.

L'analyse du produit recristallisé de l'acide acétique donne les résultats suivants :

| Analyse pour C₁₂H₁₂N₂O₂ | | | | |
|---|---|---|---|---|
| | C | H | N | O |
| Calculé | 66,65 | 5,59 | 12,95 | 14,80 |
| Trouvé | 66,55 | 5,62 | 12,76 | 15,08 |

### Etape 3

### Préparation du chlorhydrate de 6-(β-hydroxyéthyl)amino 1-méthyl indole.

On chauffe 1 heure au reflux 0,02 mole (4,32 g) de N-[6-(1-méthyl)indolyl]oxazolidine-1,3 one-2 dans 17 ml de soude 4N additionnée de 2 ml d'éthanol. Le milieu réactionnel est dilué par de l'eau glacée, puis le produit obtenu est extrait par l'acétate d'éthyle.

L'huile obtenue après évaporation de l'acétate d'éthyle est ajoutée à 7 ml d'une solution d'acide chlorhydrique 7M dans l'éthanol. Le produit attendu précipite.

L'analyse du produit obtenu après lavage et séchage, donne les résultats suivants :

| Analyse pour C₁₁H₁₅ClN₂O | | | | | |
|---|---|---|---|---|---|
| | C | H | Cl | N | O |
| Calculé | 58,28 | 6,67 | 15,64 | 12,36 | 7,06 |
| Trouvé | 58,16 | 6,70 | 15,52 | 12,45 | 7,13 |

### EXEMPLE DE PREPARATION 3

### Préparation du 6-N-(β, γ-dihydroxypropyl)amino indole.

On dissout 26,4 g de 6-amino indole dans 70 ml d'alcool absolu. On ajoute 29,6 g de glycidol et on agite pendant 4 heures à 30-40°C.

On verse sur 200 g d'eau glacée et on extrait par trois fois 100 ml d'acétate d'éthyle. On lave le solvant à l'eau. On le sèche sur Na₂SO₄ et chasse à sec sous vide.

Le résidu huileux est repris trois fois dans 0,6 litre d'éther isopropylique au reflux. On filtre l'éther et on chasse à sec sous vide. On reprend l'huile résiduelle dans 10 cm³ d'acétate d'éthyle et on purifie sur colonne de silice (éluant acétate d'éthyle 9/Heptane 1).

La fraction contenant le produit attendu est évaporée à sec sous vide.

On obtient une huile incolore qui donne les résultats suivants :

| Analyse pour C₁₁H₁₄N₂O₂ | | | | |
|---|---|---|---|---|
| | C | H | N | O |
| Calculée | 64,06 | 6,84 | 13,58 | 15,51 |
| Trouvé | 63,95 | 6,98 | 13,48 | 15,59 |

On prépare les compositions suivantes :

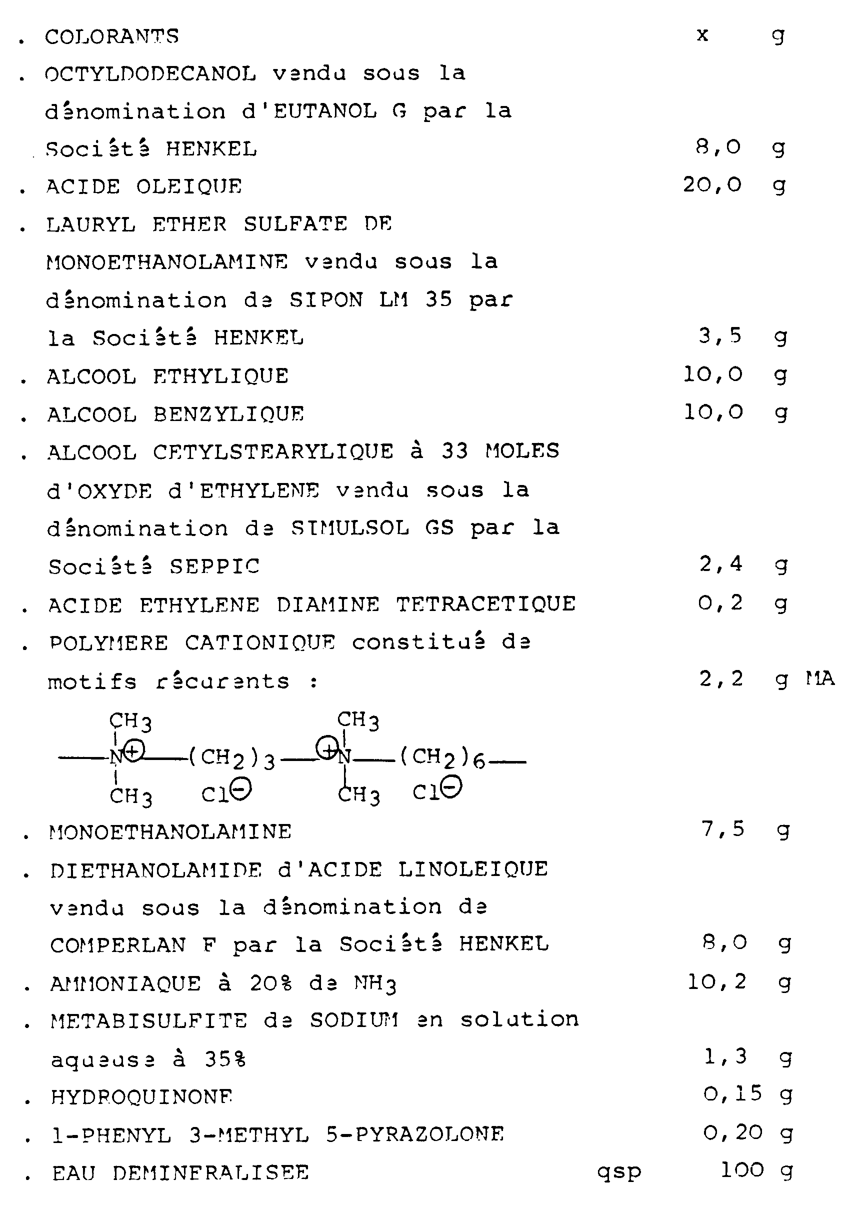

Dans tous les exemples qui suivent, les compositions sont mélangées poids par poids avec un oxydant titrant 20 volumes en eau oxygénée et dont le pH est de 3.

Les mélanges ainsi réalisés sont appliqués 30 minutes sur des cheveux gris à 90% de blancs, puis rincés et lavés. Les cheveux sont ensuite séchés.

La nature des colorants, leurs quantités et la couleur obtenue sont indiquées dans les tableaux qui suivent.

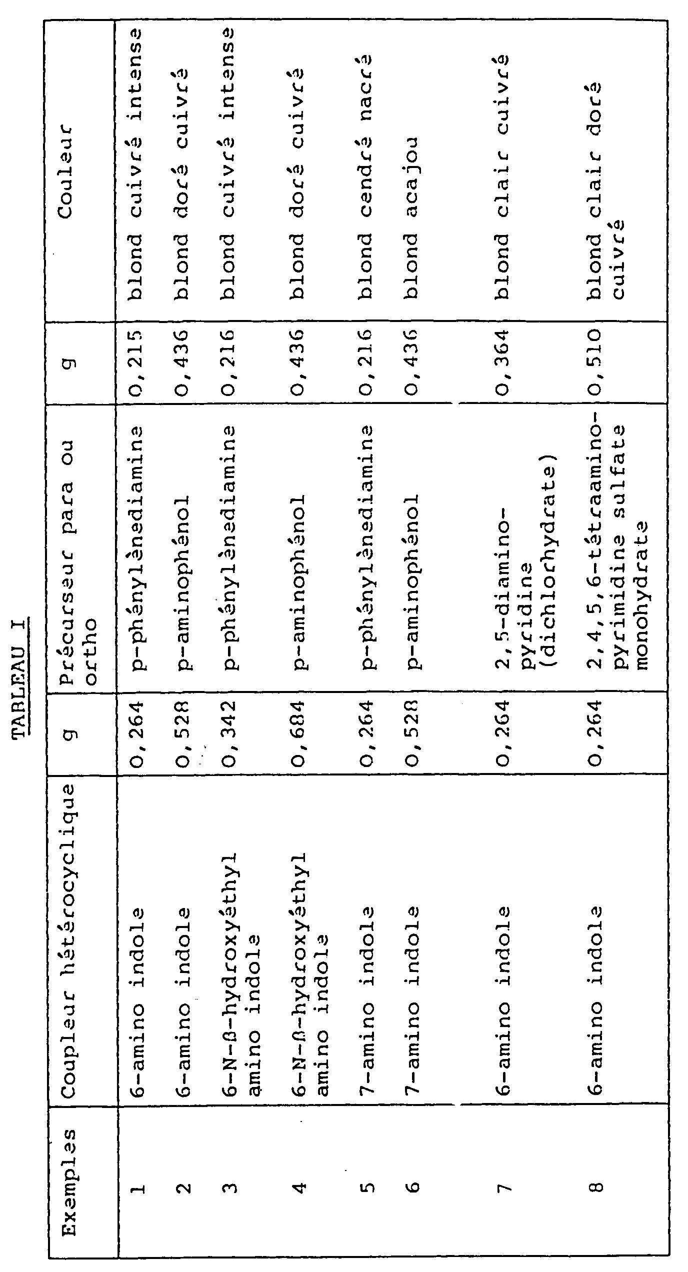

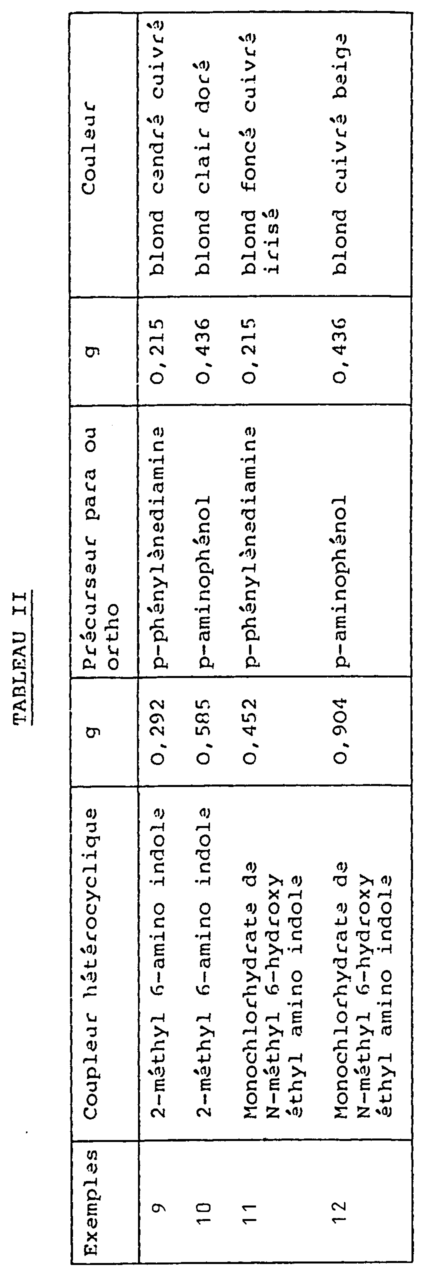

### EXEMPLE 13

On prépare le mélange tinctorial suivant :

| | |
|---|---|
| - p-phénylènediamine | 0,27 g |
| - 6-N-(β, γ-dihydroxypropyl)amino indole | 0,52 g |
| - Alcool oléique polyglycérolé à 2 moles de glycérol | 4,5 g |
| - Alcool oléique polyglycérolé à 4 moles de glycérol | 4,5 g |
| - ETHOMEEN O 12 - Société ARMOON HESS CHEMICAL Ltd (oléylamine oxyéthylénée à 12 modes d'oxyde d'éthylène) | 4,5 g |
| - COMPERLAN KD - Société HENKEL (diéthanolamide de coprah) | 9,0 g |
| - Propylène glycol | 4,0 g |
| - 2-butoxyéthanol | 8,0 g |
| - Ethanol à 96° | 6,0 g |
| - MASQUOL DTPA - Société PROTEX (sel pentasodique de l'acide diéthylène triamine pentacétique) | 2,0 g |
| - Hydroquinone | 0,15 g |
| - Solution de bisulfite de sodium à 35° Bé | 1,3 g |
| - Ammoniaque à 22° Bé | 10,0 g |
| - Eau qsp | 100,0 g |
| - pH = 9,8 | |

Au moment de l'emploi, on ajoute 10 g d'eau oxygénée à 20 volumes. Le mélange, appliqué pendant 30 minutes à 30°C sur cheveux décolorés, leur confère, après shampooing et rinçage, une coloration brun rouge.

## Revendications

1. Composition tinctoriale d'oxydation destinée à être utilisée pour la teinture des fibres kératiniques, en particulier des fibres kératiniques humaines, et notamment des cheveux, caractérisée par le fait qu'elle contient dans un milieu solvant approprie pour la teinture de ces fibres, au moins un précurseur de colorant d'oxydation para le 6-méthyl 1-hydroxy 2-aminobenzène et/ou le 4-méthyl 1-amino 2-hydroxybenzène et au moins un coupleur hétérocyclique, répondant à la formule (I) : 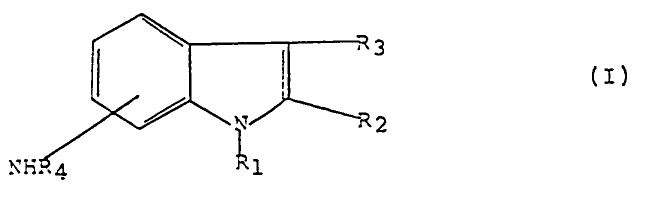 dans laquelle :
R₁ et R₃, identiques ou différents, désignent un atome d'hydrogène, un groupement alkyle en C₁-C₄;
R₂ désigne un atome d'hydrogène, un groupement alkyle en C₁-C₄, un groupement carboxyle ou un groupement alcoxycarbonyle; l'un au moins des radicaux R₂ ou R₃ désignant hydrogène;
R₄ désigne un atome d'hydrogène ou un groupement alkyle en C₁-C₄ ou hydroxyalkyle en C₁-C₄ ou polyhydroxyalkyle en C₂-C₄;
le groupement NHR₄ occupant les positions 4, 6 où 7 du noyau benzénique, ainsi que leurs sels.

2. Composition selon la revendication 1, caractérisée par le fait que les précurseurs de colorants d'oxydation para sont choisis parmi les paraphénylènediamines, les para-aminophénols, les précurseurs hétérocycliques para dérivés de la pyridine ou de la pyrimidine, les bases doubles.

3. Composition selon la revendication 2, caractérisée par le fait que les paraphénylènediamines sont choisies parmi les composes repondant à la formule (II) : 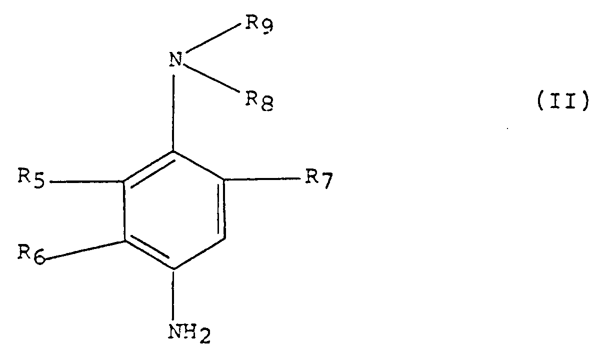 dans laquelle :
R₅, R₆ et R₇, identiques ou différents, représentent un atome d'hydrogène ou d'halogène, un radical alkyle, un radical alcoxy;
R₈ et R₉, identiques ou différents, représentent un atome d'hydrogène, un radical alkyle, hydroxyalkyle, alcoxyalkyle, carbamylalkyle, mésylamino alkyle, acétylaminoalkyle, uréidoalkyle, carbalcoxyamino alkyle, pipéridinoalkyle, morpholinoalkyle; ou bien R₈ et R₉ forment, conjointement avec l'atome d'azote auquel ils sont lies, un hétérocycle pipéridino ou morpholino, sous reserve que R₅ ou R₇ représente un atome d'hydrogène lorsque R₈ et R₉ ne représentent pas un atome d'hydrogène;
ainsi que les sels de ces composés.

4. Composition selon l'une quelconque des revendications 1 à 3, caractérisée par le fait que les coupleurs hétérocycliques de formule (I) sont choisis parmi le 6-amino indole, le 7-amino indole, le 6-N-β-hydroxyéthylamino indole, le 6-N-β-hydroxyéthylamino 1-méthyl indole, le 6-méthyl amino indole, le 6-amino N-méthyl indole, le 6-amino 2-carboxy indole, le 6-amino 3-méthyl indole, le 6-amino 2-méthyl indole, le 6-amino 2-éthoxycarbonyl indole, le 6-N-(β, γ-dihydroxypropyl) amino indole.

5. Composition selon la revendication 3, caractérisée par le fait que les paraphénylènediamines sont choisies parmi la p-phénylènediamine, la p-toluylènediamine, la méthoxyparaphénylènediamine, la 2,6-diméthyl p-phénylènediamine, la 2,5-diméthyl p-phénylènediamine, la 2-méthyl 5-méthoxyparaphénylène diamine, la 2,6-diméthyl 5-méthoxy p-phénylènediamine, la N,N-diméthylparaphénylènediamine, la 3-méthyl 4-amino N,N-diéthylaniline, la N,N-di(β-hydroxyéthyl)paraphénylènediamine, la 3-méthyl 4-amino N,N-di-(β-hydroxy éthyl)aniline, la 3-chloro 4-amino N,N-di-(β-hydroxy-éthyl)aniline, la 4-amino N,N-(éthyl,carbamylméthyl) aniline, la 3-méthyl 4-amino N,N-(éthyl,carbamylméthyl) aniline, la 4-amino N,N-(éthyl,β-pipéridinoéthyl) aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl, β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl, β-acétylaminoéthyl)aniline, la 4-amino N,N-(ethyl, β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino) phényl]morpholine, la N-[(4'-amino)phényl]pipéridine, ainsi que leurs sels.

6. Composition selon la revendication 2, caractérisée par le fait que les p-aminophénols sont choisis parmi le p-aminophénol, le 2-méthyl 4-aminophénol, le 3-méthyl 4-aminophénol, le 2-chloro 4-aminophenol, le 3-chloro 4-aminophenol, le 2,6-diméthyl 4-aminophénol, le 3,5-diméthyl 4-amino phénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-amino phénol le 3-méthoxy 4-aminophénol, le 2-méthoxyméthyl 4-aminophénol.

7. Composition selon la revendication 2, caractérisée par le fait que les bases dites doubles sont des bis-phénylalkylènediamines, répondant à la formule : 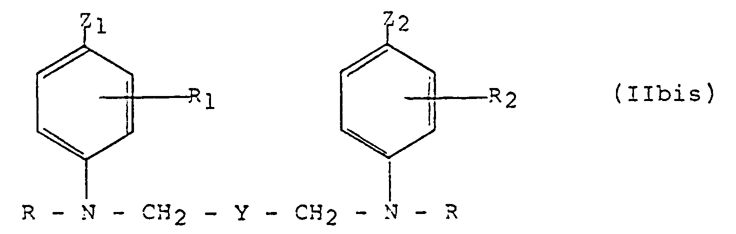 dans laquelle :
Z₁ et Z₂, identiques ou différents, représentent des groupements hydroxyle ou NHR₃, où R₃ désigne an atome d'hydrogène ou un radical alcoyle inférieur;
R₁ et R₂, identiques ou différents, représentent soit des atomes d'hydrogène, soit des atomes d'halogène, soit encore des groupements alcoyle;
R représente un atome d'hydrogène, an groupe alcoyle, hydroxyalcoyle ou aminoalcoyle, dont le reste amino peut être substitue;
Y représente un radical pris dans le groupe constitue par les radicaux suivants : -(CH₂)ₙ-, (CH₂)ₙ, -O-(CH₂)ₙ,-, -(CH₂)ₙ,-CHOH- (CH₂)ₙ,-(CH₂)ₙ, 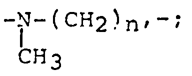 n étant un nombre entier compris entre 0 et 8 et n' un nombre entier compris entre 0 et 4, cette base pouvant se présenter sous forme de ses sels d'addition avec des acides.

8. Composition selon la revendication 7, caractérisée par le fait que les bases doubles sont choisies parmi la N,N'bis-(β-hydroxyéthyl)N,N'-bis (4'-aminophényl)1,3-diamino 2-propanol, la N,N'bis-(β-hydroxyethyl)N,N'-bis(4'-aminophenyl)éthylènediamine, la N,N'bis(4-aminophényl)tétraméthylènediamine, la N,N'bis-(β-hydroxyéthyl)N,N'bis(4-aminophényl)tétraméthylènediamine, la N,N'bis-(4-méthylaminophényl) tétraméthylènediamine, la N,N'bis(éthyl)N,N'-bis (4'-amino 3'-méthylphényl)éthylènediamine.

9. Composition selon l'une quelconque des revendications 1 à 8, caractérisée par le fait que la composition contient en plus du coupleur hétérocyclique repondant à la formule (I), d'autres coupleurs connus en eux-mêmes, choisis parmi les métadiphénols, les métaaminophénols, les métaphénylènediamines, les métaacylaminophénols, les métauréidophénols, les métacarbalcoxyaminophénols, l'α-naphtol, les coupleurs possédant un groupe méthylène actif choisis parmi les composés β-cétoniques et les pyrazolones.

10. Composition selon la revendication 9, caractérisée par le fait que le coupleur supplémentaire est choisi parmi le 2,4-dihydroxy phénoxyéthanol, le 2,4-dihydroxy anisole, le métaaminophénol, le mono méthyléther de résorcine, le 2-méthyl 5-aminophénol, le 2-méthyl 5-N-(β-hydroxyéthyl)aminophénol, le 2-méthyl 5-N-(β-mésylaminoéthyl)aminophénol, le 2,6-dimethyl 3-aminophénol, la 6-hydroxybenzomorpholine, le 2,4-diamino anisole, le 2,4-diaminophénoxyéthanol, la 6-aminobenzomorpholine, le 2-[N-(β-hydroxyéthyl)amino 4-amino]phénoxyéthanol, le 2-amino 4-N-(β-hydroxyéthyl) amino anisole, le (2,4-diamino)phényl,β, γ-dihydroxy propyléther, la 2,4-diaminophénoxyéthylamine, et leurs sels.

11. Composition selon l'une quelconque des revendications 1 à 10, caractérisée par le fait que la composition contient également des colorants directs choisis parmi les colorants azoïques, anthraquinoniques ou les dérivés nitrés de la série benzénique.

12. Composition selon l'une quelconque des revendications 1 à 11, caractérisée par le fait que l'ensemble des précurseurs de colorants par oxydation de type para ainsi que les coupleurs sont présents dans la composition dans les proportions de 0,3 à 7% en poids par rapport au poids total de la composition.

13. Composition selon l'une quelconque des revendications 1 à 12, caractérisée par le fait que la composition contient le coupleur de formule (I) dans des proportions comprises entre 0,05 et 3,5% en poids par rapport au poids total de la composition.

14. Composition selon l'une quelconque des revendications 1 à 13, caractérisée par le fait que le milieu solvant approprie pour la teinture est aqueux et que son pH est compris entre 8 et 11.

15. Composition selon l'une quelconque des revendications 1 à 14, caractérisée par le fait qu'elle contient également des agents tensio-actifs anioniques, cationiques, non ioniques, amphotères ou leurs mélanges, dans des proportions comprises entre 0,5 et 55% en poids, et de préférence entre 2 et 50% en poids par rapport au poids total de la composition.

16. Composition selon l'une quelconque des revendications 1 à 15, caractérisée par le fait que la composition contient des solvants organiques choisis parmi les alcanols inférieurs en C₁-C₄, le glycérol, les glycols ou éthers de glycols et les alcools aromatiques dans des proportions comprises entre 1 et 40% en poids.

17. Composition selon l'une quelconque des revendications 1 à 16, caractérisée par le fait qu'elle contient également des agents épaississants dans des proportions comprises entre 0,1 et 5% en poids par rapport au poids total de la composition.

18. Composition selon l'une quelconque des revendications 1 à 17, caractérisée par le fait qu'elle contient également des agents antioxydants dans des proportions comprises entre 0,05 et 1,5% en poids par rapport au poids total de la composition.

19. Procédé de teinture des fibres kératiniques, caractérisé par le fait que l'on applique sur les fibres kératiniques, en particulier les fibres kératiniques humaines et notamment les cheveux, un précurseur de colorant d'oxydation para le 6-methyl 1-hydroxy 2-aminobenzène et/ou le 4-méthyl 1-amino 2-hydroxybenzène et au moins un coupleur répondant à la formule (I) : 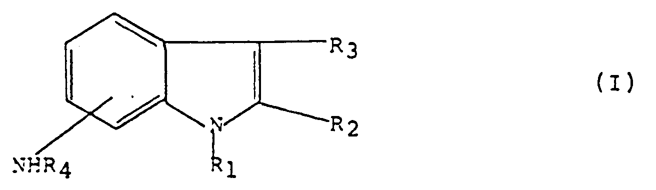 dans laquelle :
R₁ et R₃, identiques ou différents, designent un atome d'hydrogène, un groupement alkyle en C₁-C4;
R₂ désigne un atome d'hydrogène, un groupement alkyle en C₁-C₄, un groupement carboxyle ou un groupement alcoxycarbonyle;
R₄ désigne un atome d'hydrogène ou un groupement alkyle en C₁-C₄ ou hydroxyalkyle en C₁-C₄ ou polyhydroxyalkyle en C₂-C₄;
le groupement NHR₄ occupant les positions 4, 6 ou 7 du noyau benzénique, ainsi que leurs sels dans an milieu solvant approprie pour la teinture, mélangés au moment de l'emploi avec un agent oxydant en une quantite suffisante pour pouvoir développer une coloration.

20. Procédé selon la revendication 19, caractérisé par le fait que le mélange est maintenu au contact des fibres pendant 10 à 40 minutes et de préférence 15 à 30 minutes, après quoi on rince les fibres, on les lave, on les rince à nouveau et on sèche.

21. Compose caractérisé par le fait qu'il repond à la formule : 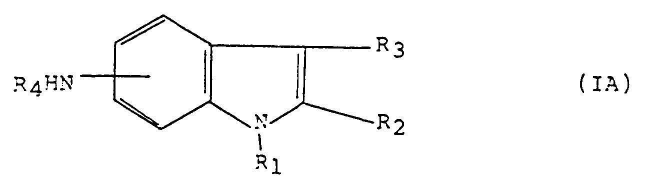 dans laquelle :
R₁ et R₃, indépendamment l'un de l'autre, représentent un atome d'hydrogène ou un groupement alkyle en C₁-C₄;
R₂ désigne hydrogène ou un groupement alkyle en C₁-C₄, un groupement carboxyle ou alcoxycarbonyle;
R₄ représente un groupement alkyle en C₁-C₄, hydroxyalkyle en C₁-C₄ ou polyhydroxyalkyle en C₂-C₄, NHR₄ occupant les positions 4, 6 ou 7 et R₄ ne peut désigner alkyle en C₁-C₄ lorsque NHR₄ est en position 4.

22. Utilisation du composé répondant à la formule : 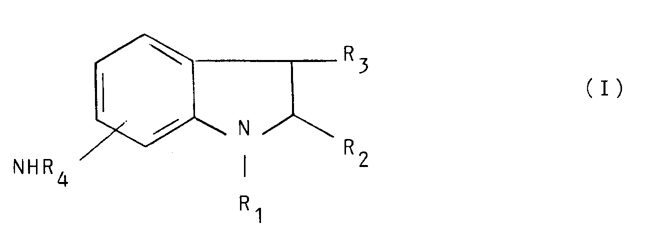 dans laquelle :
R₁ et R₃, identiques ou différents, désignent un atome d'hydrogène, un groupement alkyle en C₁-C₄;
R₂ désigne un atome d'hydrogène, un groupement alkyle en C₁-C₄, un groupement càrboxyle ou un groupement alcoxycarbonyle; l'un au moins des radicaux R₂ ou R₃ désignant l'hydrogène ;
R₄ désigne un atome d'hydrogène ou un groupement alkyle en C₁-C₄ ou hydroxyalkyle en C₁-C₄ ou polyhydroxyalkyle en C₂-C₄;
le groupement NHR₄ occupant les positions 4, 6 ou 7 du noyau benzénique, ainsi que leurs sels, à titre de coupleur dans la teinture d'oxydation de fibres kératiniques mettant en oeuvre des précurseurs de colorants d'oxydation de type para ou le 6-méthyl 1-hydroxy 2-aminobenzène et/ou le 4-méthyl 1-amino 2-hydroxybenzène.

23. Procédé de teinture des fibres kératiniques, caractérisé par le fait que l'on applique dans une première étape le précurseur de colorant d'oxydation para ou le 6-méthyl 1-hydroxy 2-aminobenzène et/ou le 4-méthyl 1-amino 2-hydroxybenzène pendant 10 à 40 minutes, puis dans une deuxième étape le coupleur de formule (I) défini dans la revendication 1, pendant 10 à 40 minutes, l'agent oxydant étant présent dans la composition appliquée dans la deuxième étape ou appliquée sur les fibres elles-mêmes au cours d'une troisième étape.

24. Composition tinctoriale d'oxydation destinée à être utilisée pour la teinture des fibres kératiniques, en particulier des fibres kératiniques humaines, et notamment des cheveux, caractérisée par le fait qu'elle contient dans un milieu solvant approprie pour la teinture de ces fibres, au moins le 6-methyl 1-hydroxy 2-aminobenzène ou le 4-méthyl 1-amino 2-hydroxybenzène et au moins un coupleur hétérocyclique, repondant à la formule (I) : 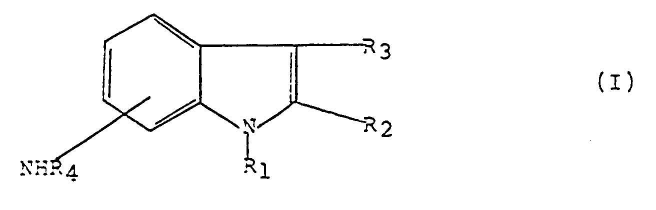 dans laquelle :
R₁ et R₃, identiques ou différents, designent un atome d'hydrogène, un groupement alkyle en C₁-C₄;
R₂ désigne un atome d'hydrogène, un groupement alkyle en C₁-C₄, un groupement carboxyle ou un groupement alcoxycarbonyle; l'un au moins des radicaux R₂ ou R₃ designant hydrogène;
R₄ désigne un atome d'hydrogène ou un groupement alkyle en C₁-C₄ ou hydroxyalkyle en C₁-C₄ ou polyhydroxyalkyle en C₂-C₄;
le groupement NHR₄ occupant les positions 4, 6 ou 7 du noyau benzénique, ainsi que leurs sels.

25. Procédé de teinture des fibres kératiniques caractérisé par le fait que l'on applique sur les fibres kératiniques, en particulier les fibres kératiniques humaines et notamment les cheveux,
au moins le 6-méthyl 1-hydroxy 2-aminobenzène ou le 4-méthyl 1-amino 2-hydroxybenzène et au moins un coupleur hétérocyclique, repondant à la formule (I) : 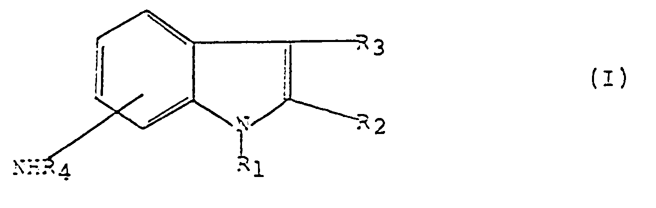 dans laquelle :
R₁ et R₃, identiques ou différents, designent un atome d'hydrogène, un groupement alkyle en C₁-C₄;
R₂ désigne un atome d'hydrogène, un groupement alkyle en C₁-C₄, un groupement carboxyle ou un groupement alcoxycarbonyle; l'un au moins des radicaux R₂ ou R₃ désignant hydrogène;
R₄ désigne un atome d'hydrogène ou un groupement alkyle en C₁-C₄ ou hydroxyalkyle en C₁-C₄ ou polyhydroxyalkyle en C₂-C₄;
le groupement NHR₄ occupant les positions 4, 6 ou 7 du noyau benzénique, ainsi que leurs sels dans un milieu solvent approprie pour la teinture, mélangés au moment de l'emploi avec un agent oxydant en une quantite suffisante pour pouvoir développer une coloration, le mélange est maintenu au contact des fibres pendant 10 à 40 minutes et de préférence 15 à 30 minutes, après quoi on rince les fibres, on les lave, on les rince à nouveau et on sèche.

## Claims

1. Oxidation tinctorial composition intended to be used for dyeing keratinous fibres, in particular human keratinous fibres and especially hair, characterized in that it contains, in a solvent medium appropriate for dyeing these fibres, at least one para oxidation dye precursor, 6-methyl-1-hydroxy-2-aminobenzene and/or 4-methyl-1-amino-2-hydroxybenzene and at least one heterocyclic coupler corresponding to the formula (I): 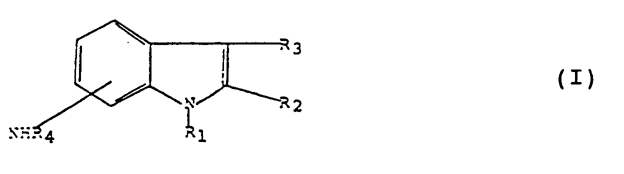 in which:
R₁ and R₃, which may be identical or different, denote a hydrogen atom or a C₁-C₄ alkyl group;
R₂ denotes a hydrogen atom, a C₁-C₄ alkyl group, a carboxyl group or an alkoxycarbonyl group; at least one of the radicals R₂ or R₃ denoting hydrogen;
R₄ denotes a hydrogen atom or a C₁-C₄ alkyl or C₁-C₄ hydroxyalkyl or C₂-C₄ polyhydroxyalkyl group;
the group NHR₄ occupying the positions 4, 6 or 7 of the benzene ring, as well as their salts.

2. Composition according to Claim 1, characterized in that the para oxidation dye precursors are chosen from para-phenylenediamines, para-aminophenols, para heterocyclic precursors derived from pyridine or pyrimidine, and double bases.

3. Composition according to Claim 2, characterized in that the para-phenylenediamines are chosen from the compounds corresponding to the formula (II): 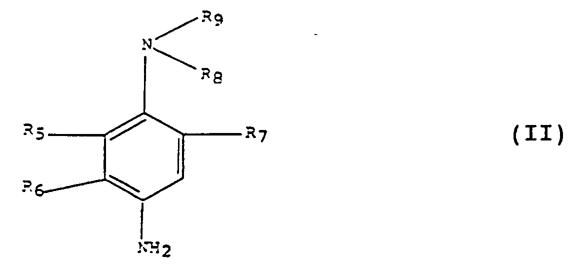 in which:
R₅, R₆ and R₇, which may be identical or different, represent a hydrogen or halogen atom, an alkyl radical or an alkoxy radical; and
R₈ and R₉, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl radical; or R₈ and R₉ form, together with the nitrogen atom to which they are bonded, a piperidino or morpholino heterocycle, with the proviso that R₅ or R₇ represents a hydrogen atom when R₈ and R₉ do not represent a hydrogen atom;
and also the salts of these compounds.

4. Composition according to any one of Claims 1 to 3, characterized in that the heterocyclic couplers of formula (I) are chosen from 6-aminoindole, 7-aminoindole, 6-N-β-hydroxyethylaminoindole, 6-N-β-hydroxyethylamino-1-methylindole, 6-methylaminoindole, 6-amino-N-methyl-indole, 6-amino-2-carboxyindole, 6-amino-3-methylindole, 6-amino-2-methylindole, 6-amino-2-ethoxycarbonylindole and 6-N(β,γ-dihydroxypropyl)aminoindole.

5. Composition according to Claim 3, characterized in that the para-phenylenediamines are chosen from p-phenylenediamine, p-toluylenediamine, methoxy-para-phenylenediamine, 2,6-dimethyl-p-phenylenediamine, 2,5-dimethyl-p-phenylenediamine, 2-methyl-5-methoxy-para-phenylenediamine, 2,6-dimethyl-5-methoxy-p-phenylenediamine, N,N-dimethyl-para-phenylenediamine, 3-methyl-4-amino-N,N-diethylaniline, N,N-di(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethylcarbamylmethyl)aniline, 3-methyl-4-amino-N,N-(ethylcarbamylmethyl)aniline, 4-amino-N,N-(ethyl-β-piperidinoethyl)aniline, 3-methyl-4-amino-N,N-(ethyl-β-piperidinoethyl)aniline, 4-amino-N,N-(ethyl-β-morpholinoethyl)aniline, 3-methyl-4-amino-N,N-(ethyl-β-morpholinoethyl)aniline, 4-amino-N,N-(ethyl-β-acetylaminoethyl)aniline, 4-amino-N-(β-methoxy-ethyl)aniline, 3-methyl-4-amino-N,N-(ethyl-β-acetylaminoethyl)aniline, 4-amino-N,N-(ethyl-β-mesylaminoethyl)aniline, 3-methyl-4-amino-N,N-(ethyl-β-mesylaminoethyl)aniline,4-amino-N,N-(ethyl-β-sulphoethyl)aniline, 3-methyl-4-amino-N,N-(ethyl-β-sulphoethyl)aniline, N-[(4'-amino)phenyl]-morpholine and N-[(4'-amino)phenyl]piperidine and their salts.

6. Composition according to Claim 2, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol and 2-methoxymethyl-4-aminophenol.

7. Composition according to Claim 2, characterized in that the so-called double bases are bis-phenylalkylenediamines corresponding to the formula: 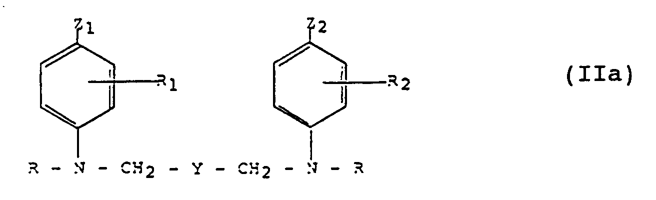 in which:
Z₁ and Z₂, which may be identical or different, represent hydroxyl or NHR₃ groups, where R₃ denotes a hydrogen atom or a lower alkyl radical;
R₁ and R₂, which may be identical or different, represent either hydrogen atoms or halogen atoms or alkyl groups;
R represents a hydrogen atom or an alkyl, hydroxyalkyl or aminoalkyl group, in which the amino radical can be substituted;
and Y represents a radical taken from the group comprising the following radicals: -(CH₂)ₙ-, (CH₂)ₙ,-O-(CH₂)ₙ,-,-(CH₂)ₙ,-CHOH-(CH₂)ₙ, 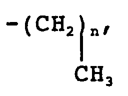 -N-(CH₂)ₙ,-; n being an integer between 0 and 8 and n' being an integer between 0 and 4, it being possible for this base to be in the form of its addition salts with acids.

8. Composition according to Claim 7, characterized in that the double bases are chosen from N,N'-bis(β-hydroxyethyl)-N,N'-bis(4'-aminophenyl)-1,3-diaminopropan2-ol, N,N'-bis(β-hydroxyethyl)-N,N'-bis(4'-aminophenyl)-ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)ethylenediamine.

9. Composition according to any one of Claims 1 to 8, characterized in that, in addition to the heterocyclic coupler corresponding to the formula (I), the composition contains other couplers known per se, such as meta-diphenols, meta-aminophenols, meta-phenylenediamines, meta-acylaminophenols, meta-ureidophenols, meta-carbalkoxyaminophenols, α-naphthol and couplers having an active methylene group, chosen from the β-ketone compounds and the pyrazolones.

10. Composition according to Claim 9, characterized in that the supplementary coupler is chosen from 2,4-dihydroxyphenoxyethanol, 2,4-dihydroxyanisole, metaaminophenol, resorcinol monomethyl ether, 2-methyl-5-aminophenol, 2-methyl-5-N-(β-hydroxyethyl)aminophenol, 2-methyl-5-N-(β-mesylaminoethyl)aminophenol, 2,6-dimethyl-3-aminophenol, 6-hydroxybenzomorpholine, 2,4-diaminoanisole, 2,4-diaminophenoxyethanol, 6-aminobenzomorpholine, 2-[N-(β-hydroxyethyl)-amino-4-amino]-phenoxyethanol, 2-amino-4-N-(β-hydroxyethyl)aminoanisole, 2,4-diaminophenyl β,γ-dihydroxypropyl ether, 2,4-diaminophenoxyethylamine and their salts.

11. Composition according to any one of Claims 1 to 10, characterized in that the composition also contains direct dyes chosen from azo or anthraquinone dyes or the nitro derivatives of the benzene series.

12. Composition according to any one of Claims 1 to 11, characterized in that the oxidation dye precursors of the para type and the couplers are present, as a whole, in the composition in proportions of from 0.3 to 7% by weight relative to the total weight of the composition.

13. Composition according to any one of Claims 1 to 12, characterized in that the composition contains the coupler of formula (I) in proportions of between 0.05 and 3.5% by weight relative to the total weight of the composition.

14. Composition according to any one of Claims 1 to 13, characterized in that the solvent medium appropriate for dyeing is aqueous and in that its pH is between 8 and 11.

15. Composition according to any one of Claims 1 to 14, characterized in that it also contains anionic, cationic, nonionic or amphoteric surfactants or their mixtures, in proportions of between 0.5 and 55% by weight, and preferably between 2 and 50% by weight, relative to the total weight of the composition.

16. Composition according to any one of Claims 1 to 15, characterized in that the composition contains organic solvents chosen from C₁-C₄ lower alkanols, glycerol, glycols or glycol ethers and aromatic alcohols in proportions of between 1 and 40% by weight.

17. Composition according to any one of Claims 1 to 16, characterized in that it also contains thickeners in proportions of between 0.1 and 5% by weight relative to the total weight of the composition.

18. Composition according to any one of Claims 1 to 17, characterized in that it also contains antioxidants in proportions of between 0.05 and 1.5% by weight relative to the total weight of the composition.

19. Method for dyeing keratinous fibres, characterized in that a para oxidation dye precursor 6-methyl-1-hydroxy-2-aminobenzene and/or 4-methyl-1-amino-2-hydroxybenzene and at least one coupler corresponding to the formula (I): 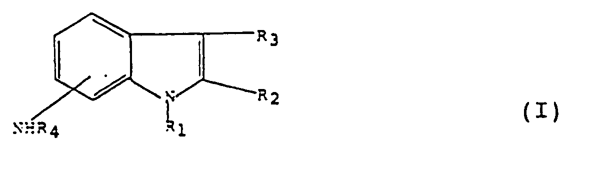 in which:
R₁ and R₃, which may be identical or different, denote a hydrogen atom or a C₁-C₄ alkyl group;
R₂ denotes a hydrogen atom, a C₁-C₄ alkyl group, a carboxyl group or an alkoxycarbonyl group;
R₄ denotes a hydrogen atom or a C₁-C₄ alkyl or C₁-C₄ hydroxyalkyl or C₂-C₄ polyhydroxyalkyl group;
the group NHR₄ occupying the positions 4, 6 or 7 of the benzene ring, as well as their salts in a solvent medium appropriate for dyeing are applied to the keratinous fibres, in particular human keratinous fibres and especially hair, said precursor and coupler being mixed at the time of use with an oxidant in an amount sufficient to be able to develop a colouring.

20. Method according to Claim 19, characterized in that the mixture is kept in contact with the fibres for 10 to 40 minutes, and preferably 15 to 30 minutes, after which the fibres are rinsed, washed, rinsed again and dried.

21. Compound, characterized in that it corresponds to the formula: 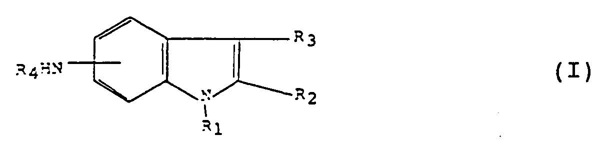 in which:
R₁ and R₃, independently of one another, represent a hydrogen atom or a C₁-C₄ alkyl group;
R₂ denotes hydrogen or a C₁-C₄ alkyl group or a carboxyl or alkoxycarbonyl group; and
R₄ represents a C₁-C₄ alkyl, C₁-C₄ hydroxyalkyl or C₂-C₄ polyhydroxyalkyl group, NHR₄ occupying the positions 4, 6 or 7 and R₄ not being able to denote C₁-C₄ alkyl when NHR₄ is in position 4.

22. Use of the compound corresponding to the formula: 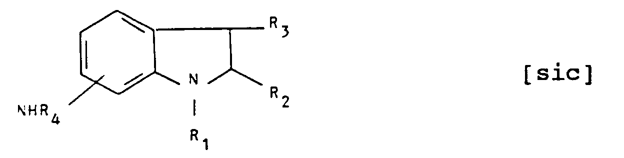 in which:
R₁ and R₃, which may be identical or different, denote a hydrogen atom or a C₁-C₄ alkyl group;
R₂ denotes a hydrogen atom, a C₁-C₄ alkyl group, a carboxyl group or an alkoxycarbonyl group; at least one of the radicals R₂ or R₃ denoting hydrogen;
R₄ denotes a hydrogen atom or a C₁-C₄ alkyl or C₁-C₄ hydroxyalkyl or C₂-C₄ polyhydroxyalkyl group;
the group NHR₄ occupying the positions 4, 6 or 7 of the benzene ring, as well as their salts, as coupler in the oxidation dyeing of keratinous fibres using oxidation dye precursors of the para type or 6-methyl-1-hydroxy-2-aminobenzene and/or 4-methyl-1-amino-2-hydroxybenzene.

23. Method for dyeing keratinous fibres, characterized in that, in a first step, the para oxidation dye precursor or 6-methyl-1-hydroxy-2-aminobenzene and/or 4-methyl-1-amino-2-hydroxybenzene are applied for 10 to 40 minutes and then, in a second step, the coupler of formula (I) defined in Claim 1 is applied for 10 to 40 minutes, the oxidant being present in the composition applied in the second step or being applied to the fibres themselves during a third step.

24. Oxidation tinctorial composition intended to be used for dyeing keratinous fibres, in particular human keratinous fibres and especially hair, characterized in that it contains, in a solvent medium appropriate for dyeing these fibres, at least 6-methyl-1-hydroxy-2-aminobenzene or 4-methyl-1-amino-2-hydroxybenzene and at least one heterocyclic coupler corresponding to the formula (I): 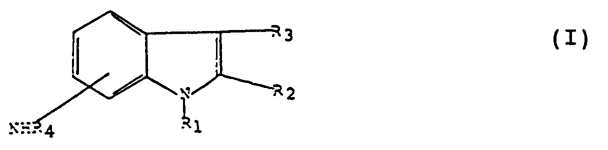 in which:
R₁ and R₃, which may be identical or different, denote a hydrogen atom or a C₁-C₄ alkyl group;
R₂ denotes a hydrogen atom, a C₁-C₄ alkyl group, a carboxyl group or an alkoxycarbonyl group; at least one of the radicals R₂ or R₃ denoting hydrogen;
R₄ denotes a hydrogen atom or a C₁-C₄ alkyl or C₁-C₄ hydroxyalkyl or C₂-C₄ polyhydroxyalkyl group;
the group NHR₄ occupying the positions 4, 6 or 7 of the benzene ring, as well as their salts.

25. Method for dyeing keratinous fibres, characterized in that at least 6-methyl-1-hydroxy-2-aminobenzene or 4-methyl-1-amino-2-hydroxybenzene and at least one heterocyclic coupler corresponding to the formula (I): 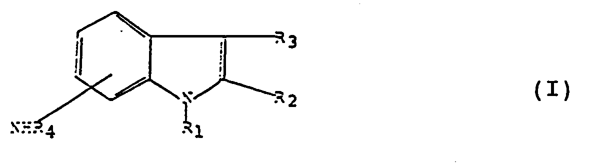 in which:
R₁ and R₃, which may be identical or different, denote a hydrogen atom or a C₁-C₄ alkyl group;
R₂ denotes a hydrogen atom, a C₁-C₄ alkyl group, a carboxyl group or an alkoxycarbonyl group; at least one of the radicals R₂ or R₃ denoting hydrogen;
R₄ denotes a hydrogen atom or a C₁-C₄ alkyl or C₁-C₄ hydroxyalkyl or C₂-C₄ polyhydroxyalkyl group;
the group NHR₄ occupying the positions 4, 6 or 7 of the benzene ring, as well as their salts in a solvent medium appropriate for dyeing are applied to the keratinous fibres, in particular human keratinous fibres and especially hair, said precursor and coupler being mixed at the time of use with an oxidant in an amount sufficient to be able to develop a colouring,
the mixture is kept in contact with the fibres for 10 to 40 minutes, and preferably 15 to 30 minutes, after which the fibres are rinsed, washed, rinsed again and dried.

## Patentansprüche

1. Oxidationsfärbezusammensetzung zur Färbung keratinischer Fasern, insbesondere menschlicher keratinischer Fasern und ganz besonders der Haare,
dadurch **gekennzeichnet,** daß
sie in einem zur Färbung dieser Fasern geeigneten Lösungsmittelmilieu mindestens eine Oxidationsfarbstoff-Vorstufe vom para-Typ, 6-Methyl-1-hydroxy-2-aminobenzol und/oder 4-Methyl-1-amino-2-hydroxybenzol und mindestens einen heterozyklischen Kuppler der Formel (I): 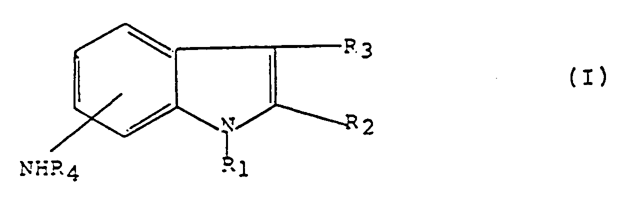 worin gilt:
R₁ und R₃ bedeuten, gleich oder verschieden, ein Wasserstoffatom oder eine C₁₋₄-Alkylgruppe;
R₂ bedeutet ein Wasserstoffatom, eine C₁₋₄-Alkylgruppe, eine Carboxyl- oder Alkoxycarbonylgruppe; wobei mindestens einer der Reste R₂ oder R₃ ein Wasserstoffatom bedeuten;
R₄ bedeutet ein Wasserstoffatom oder eine C₁₋₄-Alkyl- oder C₁₋₄-Hydroxyalkyl- oder C₂₋₄-Polyhydroxyalkylgruppe,
wobei die Gruppe NHR₄ besetzt die Positionen 4, 6 oder 7 des Benzolkerns besetzt,
sowie deren Salze enthält.

2. Zusammensetzung gemäß Anspruch 1,
dadurch **gekennzeichnet,** daß
die Oxidationsfarbstoff-Vorstufen vom para-Typ aus p-Phenylendiaminen, p-Aminophenolen, von Pyridin oder Pyrimidin abgeleiteten heterozyklischen Vorstufen vom para-Typ und aus Doppelbasen ausgewählt sind.

3. Zusammensetzung gemäß Anspruch 2,
dadurch **gekennzeichnet,** daß
die p-Phenylendiamine aus Verbindungen der Formel (II): 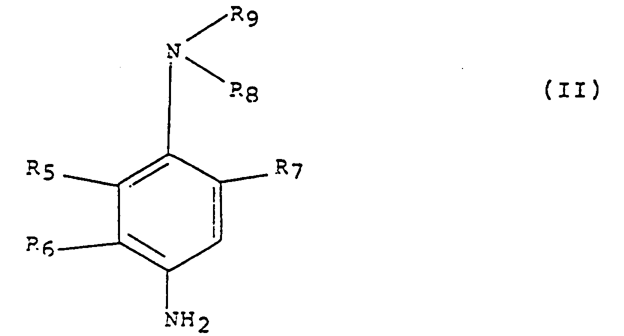 worin gilt:
R₅, R₆ und R₇ stellen, gleich oder verschieden, ein Wasserstoff- oder Halogenatom, einen Alkyl- oder Alkoxyrest dar;
R₈ und R₉ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl- oder einen Morpholinoalkylrest dar; oder R₈ und R₉ bilden, zusammen mit dem Stickstoffatom, an das sie gebunden sind, auch einen Piperidin- oder Morpholin-Heterozyklus, mit der Maßgabe, daß R₅ oder R₇ ein Wasserstoffatom darstellen, wenn R₈ und R₉ kein Wasserstoffatom darstellen;
sowie aus den Salzen dieser Verbindungen ausgewählt sind

4. Zusammensetzung gemäß jedem der Ansprüche 1 bis 3,
dadurch **gekennzeichnet,** daß
die heterozyklischen Kuppler der Formel (I) aus 6-Aminoindol, 7-Aminoindol, 6-N-β-Hydroxyethylaminoindol, 6-N-β-Hydroxyethylamino-1-methylindol, 6-Methylaminoindol, 6-Amino-N-methylindol, 6-Amino-2-carboxyindol, 6-Amino-3-methylindol, 6-Amino-2-methylindol, 6-Amino-2-ethoxycarbonylindol und aus 6-N(β,γ-Dihydroxypropyl)aminoindoi ausgewählt sind.

5. Zusammensetzung gemäß Anspruch 3,
dadurch **gekennzeichnet,** daß
die p-Phenylendiamine aus p-Phenylendiamin, p-Toluylendiamin, Methoxy-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 3-Chlor-4-amino-N,N-di(β-hydroxyethyl)anilin, 4-Amino-N,N-(ethyl, carbamylmethyl)anilin, 3-Methyl-4-amino-N,N-(ethyl, carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl, β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin und aus N-((4'-Amino)phenyl)piperidin sowie aus deren Salzen ausgewählt sind.

6. Zusammensetzung gemäß Anspruch 2,
dadurch **gekennzeichnet,** daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(β-Hydroxyethyl)4-aminophenol, 2-Methoxy-4-aminophenol, 3-Methoxy-4-aminophenol und aus 2-Methoxymethyl-4-aminophenol ausgewählt sind.

7. Zusammensetzung gemäß Anspruch 2,
dadurch **gekennzeichnet,** daß
die als doppelte bezeichneten Basen Bisphenylalkylendiamine der Formel sind: 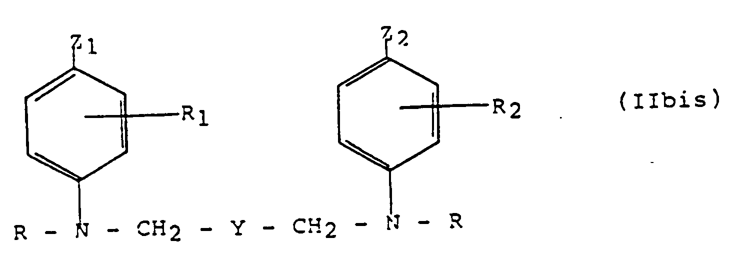 worin gilt:
Z₁ und Z₂ stellen, gleich oder verschieden, Hydroxyl- oder NHR₃-Gruppen dar, worin R₃ ein Wasserstoffatom oder eine Niedrigalkylrest bedeutet;
R₁ und R₂ stellen, gleich oder verschieden, entweder Wasserstoffatome oder Halogenatome oder auch Alkylgruppe dar; R stellt ein Wasserstoffatom, eine Alkyl-, Hydroxyalkyl- oder Aminoalkylgruppe dar, deren Aminorest substituiert sein kann; Y stellt einen Rest aus der Gruppe aus den folgenden Resten dar:
-(CH₂)ₙ-, (CH₂)_{n'}-, -O-(CH₂)_{n'}-, -(CH₂)ₙ,-CHOH-(CH₂)ₙ,, - (CH₂)ₙ,-N-CH₃-(CH₂)ₙ,
wobei n eine ganze Zahl von 0 bis 8 und n' eine ganze Zahl von 0 bis 4 sind, und wobei diese Base in Form von Additionssalzen mit Säuren vorliegen kann.

8. Zusammensetzung gemäß Anspruch 7,
dadurch **gekennzeichnet,** daß
die Doppelbasen aus N,N'-Bis(β-hydroxyethyl)-N,N'-bis(4'-aminophenyl)-1,3-diaminopropan-2-ol, N,N'-Bis(β-hydroxyethyl)-N,N'-bis(4'-aminophenyl)ethylendiamin, N,N'-Bis(4-aminophenyl)tetramethylendiamin, N,N'-Bis-(β-hydroxyethyl)-N,N'-bis(4-aminophenyl)tetramethylendiamin, N,N'-Bis-(4-methylaminophenyl)tetramethylendiamin, N,N'-Bis(ethyl)-N,N'-bis(4'-amino-3'-methylphenyl)ethylendiamin sind.

9. Zusammensetzung gemäß jedem der Ansprüche 1 bis 8,
dadurch **gekennzeichnet,** daß
die Zusammensetzung zusätzlich zum heterozyklischen Kuppler der Formel (I) weitere an sich bekannte Kuppler enthält, ausgewählt aus m-Diphenolen, m-Aminophenolen, m-Phenylendiaminen, m-Acycloaminophenolen, m-Ureidophenolen, m-Carbalkoxyaminophenolen, α-Naphtholen, Kupplern mit einer aktiven Methylengruppe, wie β-Ketoverbindungen und Pyrazolonen.

10. Zusammensetzung gemäß Anspruch 9,
dadurch **gekennzeichnet,** daß
der zusätzliche Kuppler aus 2,4-Dihydroxyphenoxyethanol, 2,4-Dihydroxyanisol, m-Aminophenol, Resorcinmonomethylether, 2-Methyl-5-aminophenol, 2-Methyl-5-N-(β-hydroxyethyl)aminophenol, 2-Methyl-5-N-(β-mesylaminoethyl)aminophenol, 2,6-Dimethyl-3-aminophenol, 6-Hydroxybenzomorpholin, 2,4-Diaminoanisol, 2,4-Diaminophenoxyethanol, 6-Aminobenzomorpholin, 2-(N-(β-Hydroxyethyl)amino-4-amino)-phenoxyethanol, 2-Amino-4-N-(β-hydroxyethyl)aminoanisol, (2,4-Diamino)phenyl-β,γ-dihydroxypropylether, 2,4-Diaminophenoxyethylamin sowie aus deren Salze ausgewählt ist.

11. Zusammensetzung gemäß jedem der Ansprüche 1 bis 10,
dadurch **gekennzeichnet,** daß
die Zusammensetzung auch Direktfarbstoffe enthält, ausgewählt aus Azofarbstoffen, Antrachinonfarbstoffen oder nitrierten Derivaten der Benzol-Reihe.

12. Zusammensetzung gemäß jedem der Ansprüche 1 bis 11,
dadurch **gekennzeichnet,** daß
die Gesamtheit der Oxidationsfarbstoff-Vorstufen vom para-Typ sowie der Kuppler in der Zusammensetzung in Mengenanteilen von 0,3 bis 7 Gew.% vorliegen, bezogen auf das Gesamtgewicht der Zusammensetzung.

13. Zusammensetzung gemäß jedem der Ansprüche 1 bis 12,
dadurch **gekennzeichnet,** daß
die Zusammensetzung den Kuppler der Formel (I) in Mengenanteilen von 0,05 bis 3,5 Gew.% enthält, bezogen auf das Gesamtgewicht der Zusammensetzung.

14. Zusammensetzung gemäß jedem der Ansprüche 1 bis 13,
dadurch **gekennzeichnet,** daß
das zur Färbung geeignete Lösungsmittelmilieu wässrig ist und sein pH-Wert 8 bis 11 beträgt.

15. Zusammensetzung gemäß jedem der Ansprüche 1 bis 14,
dadurch **gekennzeichnet,** daß
sie auch anionische, kationische, nicht-ionische oder amphotere oberflächenaktive Mittel oder deren Mischungen in Mengenanteilen von 0,5 bis 55 und vorzugsweise von 2 bis 50 Gew.% enthält, bezogen auf das Gesamtgewicht der Zusammensetzung.

16. Zusammensetzung gemäß jedem der Ansprüche 1 bis 15,
dadurch **gekennzeichnet,** daß
die Zusammensetzung organische Lösungsmittel, die aus C₁₋₄-Niedrigalkanolen, Glycerin, aus Glycolen oder Ethern von Glycolen und aus aromatischen Alkoholen ausgewählt sind, in Mengenanteilen von 1 bis 40 Gew.% enthält.

17. Zusammensetzung gemäß jedem der Ansprüche 1 bis 16,
dadurch **gekennzeichnet,** daß
sie auch Verdickungsmittel in Mengenanteilen von 0,1 bis 5 Gew.% enthält, bezogen auf das Gesamtgewicht der Zusammensetzung.

18. Zusammensetzung gemäß jedem der Ansprüche 1 bis 17,
dadurch **gekennzeichnet,** daß
sie auch Antioxidantien in Mengenanteilen von 0,05 bis 1,5 Gew.% enthält, bezogen auf das Gesamtgewicht der Zusammensetzung.

19. Verfahren zur Färbung keratinischer Fasern,
dadurch **gekennzeichnet,** daß
man auf die keratinischen Fasern, insbesondere menschliche keratinische Fasern und ganz besonders auf die Haare, eine Oxidationsfarbstoff-Vorstufe vom para-Typ, 6-Methyl-1-Hydroxy-2-aminobenzol und/oder 4-Methyl-1-amino-2-hydroxybenzol und mindestens einen Kuppler der Formel (I): 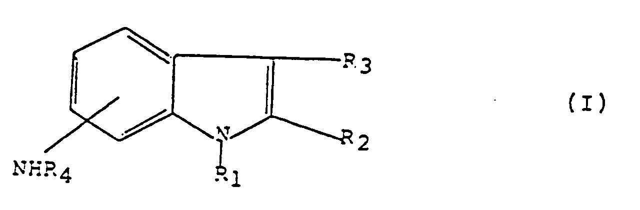 worin gilt:
R₁ und R₃ bedeuten, gleich oder verschieden, ein Wasserstoffatom oder eine C₁₋₄-Alkylgruppe;
R₂ bedeutet ein Wasserstoffatom, eine C₁₋₄-Alkylgruppe, eine Carboxyl- oder Alkoxycarbonylgruppe; wobei mindestens einer der Reste R₂ oder R₃ ein Wasserstoffatom bedeuten;
R₄ bedeutet ein Wasserstoffatom oder eine C₁₋₄-Alkyl- oder C₁₋₄-Hydroxyalkyl- oder C₂₋₄-Polyhydroxyalkylgruppe, wobei di- Gruppe NHR₄ besetzt die Positionen 4, 6 oder 7 des Benzolkens besetzt,
sowie deren Salze in einem zur Färbung geeigneten Lösungsmittelmilieu aufbringt, wobei das Ganze zum Zeitpunkt der Anwendung mit einem oxidierenden Mittel in einer zur Entwicklung einer Färbung ausreichenden Menge vermischt wird.

20. Verfahren gemäß Anspruch 19,
dadurch **gekennzeichnet,** daß
die Mischung 10 bis 40 und vorzugsweise 15 bis 30 Minuten lang in Kontakt mit den Fasern gehalten wird, worauf man die Fasern spült, sie wäscht, sie erneut spült und trocknet.

21. Verbindung,
dadurch **gekennzeichnet,** daß
sie die Formel aufweist: 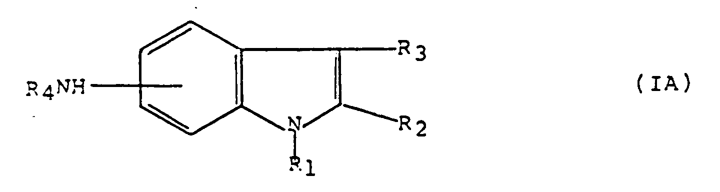 worin gilt:
R₁ und R₃ stellen, unabhängig voneinander, ein Wassserstoffatom oder eine C₁₋₄-Alkylgruppe dar;
R₂ bedeutet ein Wasserstoffatom oder eine C₁₋₄-Alkyl-, Carboxyl- oder Alkoxycarbonylgruppe;
R₄ stellt eine C₁₋₄-Alkyl-, C₁₋₄-Hydroxyalkyl- oder C₂₋₄-Polyhydroxyalkylgruppe dar, wobei NHR₄ die Positionen 4, 6 oder 7 besetzt und R₄ keine C₁₋₄-Alkylgruppe bedeutet, wenn NHR₄ in Position 4 vorliegt.

22. Verwendung der Verbindung der Formel: 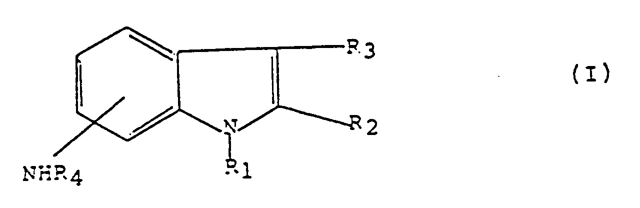 worin gilt:
R₁ und R₃ bedeuten, gleich oder verschieden, ein Wasserstoffatom oder eine C₁₋₄-Alkylgruppe;
R₂ bedeutet ein Wasserstoffatom, eine C₁₋₄-Alkylgruppe, eine Carboxyl- oder Alkoxycarbonylgruppe; wobei mindestens einer der Reste R₂ oder R₃ ein Wasserstoffatom bedeuten;
R₄ bedeutet ein Wasserstoffatom oder eine C₁₋₄-Alkyl- oder C₁₋₄-Hydroxyalkyl- oder C₂₋₄-Polyhydroxyalkylgruppe,
wobei die Gruppe NHR₄ besetzt die Positionen 4, 6 oder 7 des Benzolkerns besetzt,
sowie von deren Salzen als Kuppler in der Oxidationsfärbung keratinischer Fasern, wobei man Oxidationsfarbstoff-Vorstufen vom para-Typ oder 6-Methyl-1-hydroxy-2-aminobenzol und/oder 4-Methyl-1-amino-2-hydroxybenzol zur Anwendung bringt.

23. Verfahren zur Färbung keratinischer Fasern,
dadurch **gekennzeichnet,** daß
man in einer ersten Stufe die Oxidationsfarbstoff-Vorstufe vom para-Typ oder 6-Methyl-1-hydroxy-2-aminobenzol und/oder 4-Methyl-1-amino-2-hydroxybenzol 10 bis 40 Minuten lang und dann in einer zweiten Stufe den in Anspruch 1 definierten Kuppler der Formel (I) 10 bis 40 Minuten lang aufbringt, wobei das oxidierende Mittel in der in der zweiten Stufe aufgebrachten Zusammensetzung vorhanden ist oder auf die Fasern selbst während einer dritten Stufe aufgebracht wird.

24. Oxidationsfärbezusammensetzung zur Färbung keratinischer Fasern, insbesondere menschlicher keratinischer Fasern und ganz besonders der Haare,
dadurch **gekennzeichnet,** daß
sie in einem zur Färbung dieser Fasern geeigneten Lösungsmittelmilieu mindestens 6-Methyl-1-hydroxy-2-aminobenzol oder 4-Methyl-1-amino-2-hydroxybenzol und mindestens einen heterozyklischen Kuppler der Formel (I): 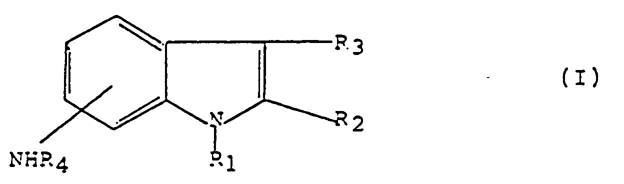 worin gilt:
R₁ und R₃ bedeuten, gleich oder verschieden, ein Wasserstoffatom oder eine C₁₋₄-Alkylgruppe;
R₂ bedeutet ein Wasserstoffatom, eine C₁₋₄-Alkylgruppe, eine Carboxyl- oder Alkoxycarbonylgruppe; wobei mindestens einer der Reste R₂ oder R₃ ein Wasserstoffatom bedeuten;
R₄ bedeutet ein Wasserstoffatom oder eine C₁₋₄-Alkyl- oder C₁₋₄-Hydroxyalkyl- oder C₂₋₄-Polyhydroxyalkylgruppe, wobei die Gruppe NHR₄ besetzt die Positionen 4, 6 oder 7 des Benzolkerns besetzt,
sowie deren Salze enthält.

25. Verfahren zur Färbung keratinischer Fasern,
dadurch **gekennzeichnet,** daß
man auf die keratinischen Fasern, insbesondere auf menschliche keratinische Fasern und ganz besonders auf die Haare, mindestens 6-Methyl-1-hydroxy-2-aminobenzol oder 4-Methyl-1-amino-2-hydroxybenzol und mindestens einen heterozyklischen Kuppler der Formel (I): 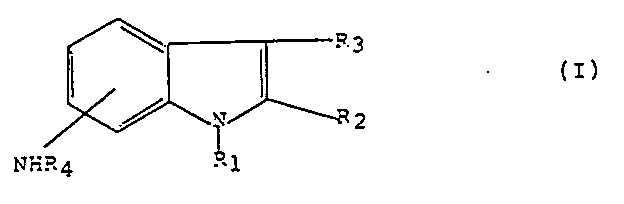 worin gilt:
R₁ und R₃ bedeuten, gleich oder verschieden, ein Wasserstoffatom oder eine C₁₋₄-Alkylgruppe;
R₂ bedeutet ein Wasserstoffatom, eine C₁-₄-Alkylgruppe, eine Carboxyl- oder Alkoxycarbonylgruppe; wobei mindestens einer der Reste R₂ oder R₃ ein Wasserstoffatom bedeuten;
R₄ bedeutet ein Wasserstoffatom oder eine C₁₋₄-Alkyl- oder C₁₋₄-Hydroxyalkyl- oder C₂₋₄-Polyhydroxyalkylgruppe,
wobei die Gruppe NHR₄ besetzt die Positionen 4, 6 oder 7 des Benzolkerns besetzt,
sowie deren Salze in einem zur Färbung geeigneten Lösungsmittelmilieu aufbringt, wobei das Ganze zum Zeitpunkt der Anwendung mit einem oxidierenden Mittel in einer zur Entwicklung einer Färbung ausreichenden Menge vermischt wird, und wobei die Mischung 10 bis 40 und vorzugsweise 15 bis 30 Minuten lang in Kontakt mit den Fasern gehalten wird, worauf man die Fasern spült, sie wäscht, erneut spült und trocknet.
